(19) Europäisches Patentamt
European Patent Office
Office européen des brevets

(11) **EP 2 037 236 A2**

(12) **EUROPÄISCHE PATENTANMELDUNG**

(43) Veröffentlichungstag:
**18.03.2009 Patentblatt 2009/12**

(51) Int Cl.:
***G01F 25/00*** *(2006.01)* ***A61M 1/10*** *(2006.01)*

(21) Anmeldenummer: **08159155.4**

(22) Anmeldetag: **27.06.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **11.09.2007 EP 07116095**

(71) Anmelder: **Levitronix LLC**
**Waltham, MA 02451 (US)**

(72) Erfinder:
• **Fleischli, Andreas**
  **6340, Baar (CH)**
• **Gempp, Thomas, Dr.**
  **8964, Rudolfstetten (CH)**

(74) Vertreter: **Sulzer Management AG**
**Patentabteilung / 0067**
**Zürcherstrasse 14**
**8401 Winterthur (CH)**

(54) **Verfahren zur Kalibrierung einer Durchflussmessung in einem Strömungssystem, sowie ein Strömungssystem zur Durchführung des Verfahrens**

(57)  Die Erfindung betrifft ein Verfahren zur Kalibrierung einer Durchflussmessung in einem Strömungssystem (1), wobei ein Fluid (2) durch eine Strömungsverbindung (3) des Strömungssystems (1) befördert wird, und durch die Durchflussmessung eine Durchflussmenge des Fluids (2) aus einem Arbeitsparameter des Strömungssystems (1) bestimmt wird. Erfindungsgemäss wird dabei zu einem vorgegebenen Zeitpunkt mit einem Kalibriersensor (4) die Durchflussmenge des Fluids (2) in einer Kalibiermessung ermittelt, und die Durchflussmessung anhand der Kalibriermessung neu kalibriert. Darüber hinaus betrifft die Erfindung ein Strömungssystem zur Durchführung des erfindungsgemässen Verfahrens.

Fig.3

EP 2 037 236 A2

**Beschreibung**

Die Erfindung betrifft ein Verfahren zur Kalibrierung einer Durchflussmessung in einem Strömungssystem, sowie ein Strömungssystem zur Durchführung des Verfahrens gemäss dem Oberbegriff des unabhängigen Anspruchs der jeweiligen Kategorie.

Zur Durchflussmessung, also zur Bestimmung derjenigen Menge eines Fluids, die pro Zeiteinheit durch ein Strömungssystem befördert wird, sind inzwischen eine kaum überblickbare Zahl von Methoden und Anwendungen bekannt.

Gemessen werden Durchflussmengen von Flüssigkeiten, zum Beispiel in Einspritzsystemen von Brennkraftmaschinen, wie z.B. bei Motoren von Kraftfahrzeugen, Flugzeugen oder Schiffen, Durchflussmengen von Rohöl durch eine Pipeline müssen kontinuierlich, z.B. zu Abrechnungszwecken gemessen und überwacht werden. Beim Befüllen von Behältnissen müssen Füllmengen gemessen und dosiert werden usw..

Auch Durchflussmengen von gasförmigen Fluiden, wie z.B. von Erdgas durch eine Pipeline oder zu Abrechnungszwecken beim Betanken von Kraftfahrzeugen werden heute durch zu Hilfenahme verschiedenster Systeme standardmässig bestimmt.

Dabei steht zur Bestimmung der Durchflussmenge eine grosse Zahl physikalischer Grössen und Eigenschaften des Fluids bzw. des strömenden Systems zur Verfügung, durch deren Messung die Durchflussmenge des Fluids durch das Strömungssystem bestimmt werden kann.

Welche Grösse im Speziellen zur Messung der Durchflussmenge im konkreten Fall herangezogen wird, kann von vielen Faktoren abhängen. Zum einen natürlich davon, ob das Fluid flüssig oder gasförmig, oder im speziellen Fall sogar fest ist, wie zum Beispiel beim Strömen eines feinen abrasiven Sandes in einem Sandstrahlgerät oder eines feinen Spritzpulvers in einem thermischen Spritzgerät. Je nach dem sind dann gewisse Grössen von vorneherein geeignet oder nicht geeignet. So müssen z.B. bei der Bestimmung der Durchflussmenge eines Gases spezielle Anstrengungen unternommen werden, da z.B. die Messung des pro Zeiteinheit durchströmenden Volumens durch das Strömungssystem nur begrenzt aussagen über die tatsächliche Menge, also die Masse des transportieren Gases liefert, da diese bei gegebenem Volumenstrom sehr stark z.B. von der Temperatur und dem Druck des Gases abhängt.

Beim strömenden Transport flüssiger Stoffe spielen dagegen Grössen wie Druck und Temperatur des Fluids häufig keine Rolle, so dass zumindest dann, wenn keine all zu grosse Präzision gefordert ist, eine Messung des pro Zeiteinheit durch das Strömungssystem durchströmenden Volumens völlig ausreichend ist, um auf die tatsächlich geflossene Stoffmenge, also z.B. auf die transportierte Masse des flüssigen Fluids zu fliessen.

Oftmals ist es somit möglich und auch angebracht, einfache Sensoren wie z.B. Flügelräder, Schwebekörper, Blenden zur Messung nach dem Differenzdruckverfahren, Venturidüsen im Fall von Gasen und viele andere, dem Fachmann seit langen wohl bekannte Messvorrichtungen direkt innerhalb einer Strömungsverbindung des Strömungssystems vorzusehen, so dass das strömende Fluid in unmittelbaren wechselwirkenden Kontakt mit der entsprechenden Messvorrichtung, z.B. mit dem Flügelrad treten kann, so dass, beispielsweise aus der Drehgeschwindigkeit des Flügelrads die Durchflussmenge unmittelbar ableitbar ist.

Somit stehen in einfachen Fällen, d.h. wenn keine all zu grossen Anforderungen an die Präzision der Durchflussmessung gestellt sind, und /oder wenn z.B. keine. hygienisch oder technisch sensiblen Flüssigkeiten, wie Blut, pharmazeutische oder chemisch hochreine Fluide, oder hochreine Flüssigkeiten in der Halbleiterindustrie, wie z.B. Slurries zum Polieren von Halbleiter Wafern, gemessen werden müssen, eine Vielzahl von Messsystemen und Methoden zur Auswahl, die zum Teil beliebig austauschbar sind und den technischen Anforderungen in Bezug auf Präzision, Langzeitstabilität und chemische bzw. physikalische Kompatibilität mit dem zu messenden Fluid vollauf genügen.

Grössere Anstrengungen müssen jedoch unternommen werden, wenn die Anforderungen an die Durchflussmessung steigen. Zum Beispiel können auch bei der Messung von Flüssigkeiten Temperaturschwankungen oder Druckänderungen das Messergebnis beeinflussen, wenn eine entsprechende Präzision der Ergebnisse gefordert ist. Je nach Messmethode können auch andere Grössen, wie zum Beispiel die Viskosität des Fluids, die sich im Laufe des Messprozesses verändern kann, die Ergebnisse in unzulässiger Weise beeinflussen.

Auch ist es in speziellen Anwendungen von grösster Bedeutung, dass die Messung möglichst nicht invasiv erfolgt. Das heisst, es ist oft wesentlich, dass die Messeinrichtung als solches nicht in direkten körperlichen Kontakt mit dem zu messenden Fluid kommt. Das kann z.B. dann von Bedeutung sein, wenn das Fluid mit dem entsprechenden Messkörper z.B. chemisch oder physikalisch nicht kompatibel ist, weil beispielsweise das Fluid chemisch, wie im Fall einer starken Säure oder Lauge, sehr aggressiv ist oder aber auch physikalisch aggressiv ist, wie beispielweise das oben bereits erwähnte Slurry, das hoch abrasiv wirkt und einen Messkörper, wie zum Beispiel einen Schwebekörper oder ein Flügelrad in kürzester Zeit zerstören könnte.

Aber nicht nur die Messapparatur kann durch das zu messende Fluid negativ beeinträchtigt werden. Ungekehrt kann in speziellen Fällen auch die Messapparatur negativ auf das zu messende Fluid rückwirken.

So müssen bei sensiblen Flüssigkeiten wie Blut oder hoch reinen pharmazeutischen oder chemischen Produkte unbedingt Verunreinigungen vermieden werden. Diese können zum Beispiel bei einem mechanischen Lager, in dem ein Flügelrad zur Messung der Durchflussmenge gelagert ist, in Form von Lagerschmierstoffen oder mechanischem Abrieb zu Verunreinigungen des Fluids im Strömungssystem führen.

Beispielsweise Blut ist darüber hinaus eine Flüssigkeit, die auch auf mechanische Einwirkungen ausgesprochen sensibel reagiert. Ist das Strömungssystem ein Ventrikel Unterstützungssystem zur Unterstützung eines menschlichen oder tierischen Blutkreislaufs, also ein Strömungssystem aus Blutpumpe, Kanülen und Zuleitungen, welches zum Beispiel während einer Operationen einen Bypass für das Herz bildet oder in einer Langzeitanwendung ein geschwächtes Herz entlastet, so ist die Durchflussmenge des Blues eine sehr kritische Systemgrösse, die ständig überwacht und eventuell durch geeignete Steuerung der Blutpumpe sensibel nachgeregelt werden muss.

In einem solchen Strömungssystem ist die Messung der Durchflussmenge des Blutes in Bezug auf verschiedene Aspekte eine besonders kritische Herausforderung.

Kommt es zum Beispiel zu Engstellen und / oder zu einer lokal starken mechanischen Belastung im Ventrikel Unterstützungssystem, z.B. zu einer Kompression des Bluts zwischen einem Flügelrad einer Strömungsmesskomponente und einer Wand der Strömungsverbindung, so kann eine Zerstörung oder Quetschung der roten Blutkörperchen resultieren oder es kann zu Anlagerungen und / oder Ablagerungen von Blut am Strömungsmesskörper, z.B. an einem Flügelrad zur Strömungsmessung kommen und im schlimmsten Fall sogar zu Verklumpungen des Bluts führen, was zu schweren Schädigungen des Patienten, wie Thrombosen, Gefässverschlüssen und sogar zum Infarkt, im schlimmsten Fall zum Tod des Patienten führen kann.

Daher werden in solchen medizinischen Systemen, aber auch in rein technischen Systemen, z.B. für die Halbleiterindustrie, wo wie oben beschrieben sehr hohe Reinheit gefordert ist, bevorzugt keine zusätzlichen invasiven Messapparaturen im Strömungssystem vorgesehen um die Durchflussmengen des zu befördernden Fluids zu kontrollieren.

Man versucht vielmehr die Durchflussmengen aus ohnehin verfügbaren Betriebsdaten des Systems abzuleiten. Enthält ein solches System z.B. eine Pumpe, wie bei einem Ventrikel Unterstützungssystem zur Unterstützung bzw. kurzzeitigen Ersetzung der Funktion des Herzens eines Patienten, so können die Durchflussmengen zum Beispiel durch Auswertung der Betriebsdaten des elektrischen Pumpenmotors abgeleitet werden.

Dazu werden elektrische Betriebsdaten des Motors, z.B. die Aufnahme elektrischer Energie durch den Pumpenmotor, im Speziellen der vom Pumpenmotor aufgenommene elektrische Strom, wobei insbesondere der Antriebsstrom und / oder die Drehzahl des Rotors in einem für die Praxis besonders wichtigen Ausführungsbeispiel massgebliche Eingangsgrössen für die Berechnung des Durchflusses sind, und die ständig überwacht und registriert werden und daraus, z.B. unter zu Hilfenahme einer Look-Up-Tabelle oder anderer Eichverfahren die Durchflussmengen ermittelt werden.

Dieses Verfahren hat sich vor allem beim Einsatz sogenannter lagerloser Motoren, wie sie später noch eingehender beschrieben werden, bewährt, weil bei diesem Typ von Motoren die Durchflussmengen sehr einfach und zuverlässig aus den elektrischen Betriebsdaten des Motors abgeleitet werden können. Es sind dann keine zusätzlichen apparativen Elemente zur Messung des Durchflusses notwendig, die invasiv mit dem zirkulierenden Blut, oder im Fall anderer nicht medizinischer Strömungssysteme mit anderen Fluiden wechselwirken.

Im Folgenden soll kurz ein Grundkonzept für die Bestimmung des Durchflusses aus den entsprechenden Leistungskurven der Pumpe bzw. des Pumpenantriebs erläutert werden.

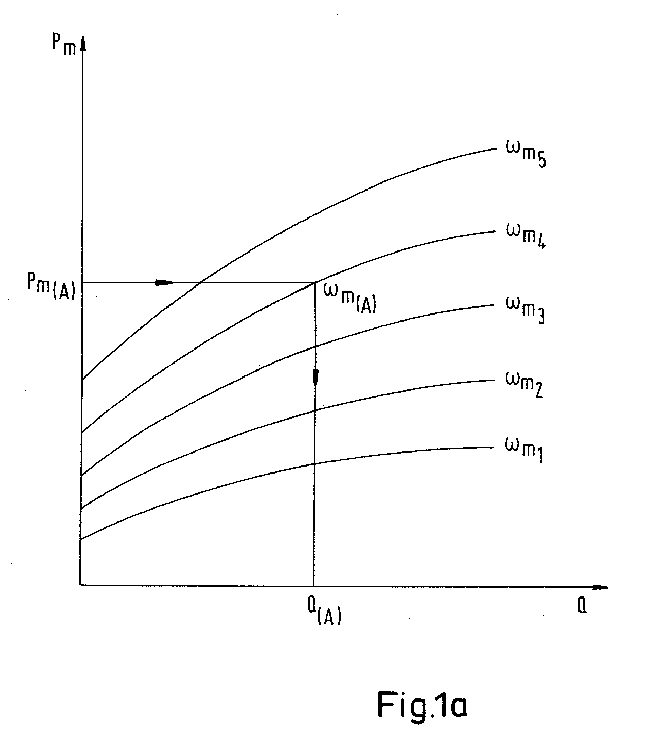

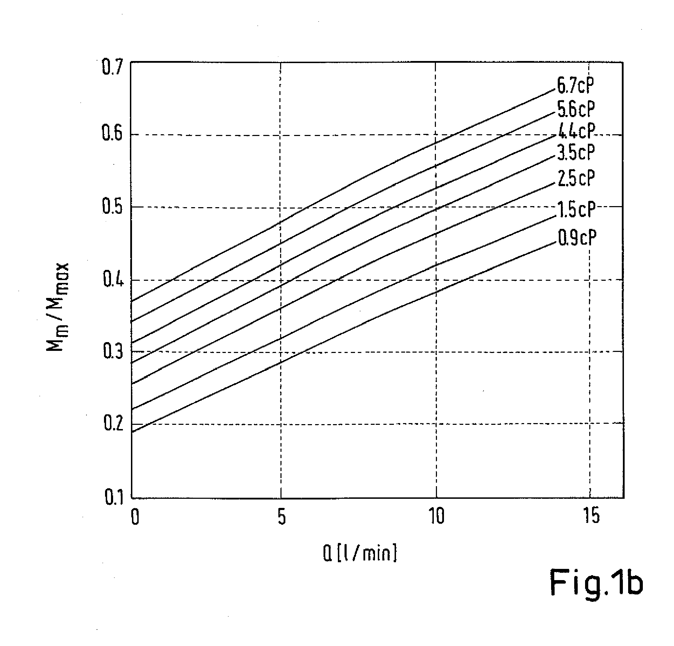

Die verwendeten Formelzeichen, wie sie teilweise auch in den Fig. 1a und 1b zu finden sind, haben dabei wie gewöhnlich die folgende Bedeutung: $P_m$ ist die am Rotor abgegebene mechanische Leistung; M ist das totale Motordrehmoment; $M_m$ ist das am Rotor abgegebene mechanische Drehmoment; $M_L$ ist das hydraulische Lastmoment, $M_l$ ist das Leerlaufdrehmoment; $\omega_m$ ist die mechanische Kreisfrequenz, die direkt proportional zur Drehzahl des Rotors ist; Q ist der durch die Pumpe fliessende Durchfluss; $I_M$ ist der Drehmomentstrom; $\gamma_m$ ist der mechanische Rotorwinkel; und $c_M$ ist eine Drehmomentkonstante.

Die Leistungskurven geben den Zusammenhang zwischen mechanischer Leistung $P_m$ und hydraulischer Leistung, das dem Durchfluss Q in einer Pumpe an. Sie sind, wie in Fig. 1a schematisch dargestellt, eine Kurvenschar, wobei die Parametrisierung mit der Drehzahl $\omega$ des Rotors erfolgt. Jede einzelne Kurve stellt den Zusammenhang zwischen am Rotor abgegebener mechanischer Leistung $P_m$ und dem Durchfluss durch die Pumpe bei konstanter Drehzahl $\omega_m$ dar. Die mechanische Leistung kann nach der Bestimmung von Drehmoment $M_m$ und Drehzahl $\omega_m$ berechnet werden:

$$P_m = M_m \cdot \omega_m$$

Sind die Pumpenkennlinien bekannt, kann aus den Leistungskurven unter Berücksichtigung der berechneten mechanischen Leistung und der Drehzahl des Rotors der Durchfluss durch die Pumpe ermittelt werden.

Bei konstanter Drehzahl ist das Drehmoment $M_m$ direkt proportional zur mechanischen Leistung $P_m$. Aufgrund

der reibungsfreien magnetischen Lagerung entspricht das mechanische Moment $M_m$ direkt dem Hydraulischen Lastmoment $M_L$.

z.B. im Fall eines UltraMag Systems, das die Anmelderin vermarktet, erzielt man mit einem im Durchfluss linearen Interpolationsansatz für die Leistungskurven eine ausreichende Genauigkeit.

$$M_m = M_L = c^{(P1)}(\omega_m) \cdot Q + c^{(P0)}(\omega_m)$$

Die Koeffizienten $c^{(P0)}$ und $c^{(P1)}$ sind von der Drehzahl des Rotors abhängig:

$$c^{(P0)}(\omega_m)= c_2^{(P0)} \cdot \omega_m{}^2 + c_1^{(P0)} \cdot \omega_m$$

$$c^{(P1)}(\omega_m)= c_1^{(P1)} \cdot \omega_m$$

Daraus ergibt sich:

$$M_m = M_L =( c_1^{(P1)} \cdot \omega_m) \cdot Q + c_2^{(P0)} \cdot \omega_m{}^2 + c_1^{(P0)} \cdot \omega_m$$

Und daraus ergibt sich der Durchfluss Q:

$$Q=(M_m - ( c_2^{(P0)} \cdot \omega_m{}^2 + c_1^{(P0)} \cdot \omega_m )) / ( c_1^{(P1)} \cdot \omega_m )$$

Im Folgenden soll auch noch auf die Drehmomentberechnung und die Drehzahlberechnung etwas detaillierter eingegangen werden.

Für die Berechnung des Durchflusses wird das Drehmoment M benötigt, das sich aus der Drehmomentkonstante $c_m$ des Motors und dem drehmomentbildenden Momentenstrom $I_M$ des Motors berechnet.

$$M = c_M \cdot I_M$$

Dieses Drehmoment M setzt sich zusammen aus dem Leerlaufdrehmoment $M_l$, das die inneren Verluste des Motors abdeckt, und dem am Rotor abgegebenem mechanischen Drehmoment $M_m$. Daraus ergibt sich:

$$M = M_l + M_m$$

Sowohl die Drehmomentkonstante $c_m$ wie auch das Leerlaufdrehmoment $M_l$ sind drehzahlabhängig. Daraus ergibt sich:

$$M_m = c_M(\omega_m) \cdot I_M - M_l(\omega_m)$$

Der Drehmomentstrom wird gemessen und ist im System bekannt.

Zur Drehzahlberechnung wird der Rotorwinkel $\gamma_m$ wird über eine Feldmessung mit Hallsonden bestimmt. Die Ableitung des Rotorwinkels $\gamma_m$ ergibt die Drehzahl $\omega_m$:

$$\omega_m = d\gamma_m / dt$$

Als alternative Methode kann die Drehzahl $\omega_m$ auch aus der induzierten Spannung der Antriebsphasen ermittelt werden, was aber nicht zwingend implementiert werden muss.

Einen entscheidenden Einfluss hat dabei auch das Fördermedium, also zum Beispiel das Fördermedium Blut. Die Leistungskurve der Blutpumpe ist nämlich auch von der Dichte und der Viskosität des Blutes abhängig.

Die Dichte des Blutes ändert sich in der Praxis meist nur gering und darf in vielen Fällen als erste Näherung als konstant angesehen werden.

Die Blutviskosität variiert dagegen beträchtlich und steht, wie an sich dem Fachmann bekannt, in Abhängigkeit verschiedener Parameter, wie Hämatokrit, männlich/weiblich, Raucher/Nichtraucher, Temperatur, Cholesterin-Spiegel, Körper-Fitness-Index, usw..

Im Normalfall befindet sich der Hämatokritwert im Bereich von 35 - 50%. Werden auch Extremfälle berücksichtigt, muss mit einem Hämatokritbereich zwischen 15 und 60% gerechnet werden. Gemäss "Jürgen Hahn, Sensorlose Bestimmung der Prozessgrössen magnetisch gelagerter Blutpumpen, Dissertation ETH Zürich, Nr. 14563, Zürich 2002", deren Inhalt hiermit in diese Anmeldung inkorporiert ist, wird der folgende, in Tabelle 1 dargestellte Zusammenhang zwischen Hämatokrit und der Viskosität aufgezeigt:

| HCT in % | Dynamische Viskosität in cP | | |
|---|---|---|---|
| 15 | 2.05 | | |
| 35 | 2.89 | | |
| 40 | 3.26 | Normal- | Erweiteter |
| 45 | 3.74 | bereich | Bereich |
| 50 | 4.36 | | |
| 60 | 6.1 | | |

Tabelle 1: Zusammenhang zwischen Hämatokrit und Viskosität

Das zeigt, dass die Viskosität alleine aufgrund des variierenden Hämatokrit beträchtlich variiert.

Die Theorie und die Praxis zeigt, dass die dynamischen Viskositätskoeffizienten $C_2^{(P0)}$ und $c_1^{(P0)}$ der in der Gleichung

$$M_m = M_L = ( c_1^{(P1)} \cdot \omega_m ) \cdot Q + c_2^{(P0)} \cdot \omega_m{}^2 + c_1^{(P0)} \cdot \omega_m$$

beschriebenen Leistungskurven ist von der Viskosität der geförderten Flüssigkeit abhängig.

Die Fig. 1b zeigt dazu die Leistungskurven bei Blutersatzflüssigkeiten unterschiedlicher Viskosität bei einer konstanten Drehzahl. Aufgetragen ist das Verhältnis von maximalem mechanischem Drehmoment $M_{max}$ zu dem am Rotor abgegebenen mechanischen Drehmoment $M_m$ gegen die Durchflussmenge Q in Liter pro Minute. Mit steigender Viskosität erhöht sich der Leistungsbedarf und die Leistungskurven verschieben sich parallel nach oben. Das heisst, im wesentlichen wird nur der Offsetwert der Kurven wird von der Viskosität beeinflusst.

Durch Anwendung dieser speziellen, an sich bekannten Technik kann somit der Durchfluss des Fluids durch ein Strömungssystem bestimmt werden, ohne dass es zu negativen Auswirkungen bzw. Einwirkungen auf das Fluid kommt.

Es hat sich jedoch gezeigt, dass auch bei diesen Systemen zumindest dann, wenn höchste Anforderungen an die Präzision der Durchflussmessung bzw. an die Betriebssicherheit des Systems insgesamt gestellt sind, noch erheblicher Verbesserungsbedarf besteht.

So hängt beispielsweise die aus den elektrischen Betriebsdaten ermittelte Durchflussmenge des Fluids in bestimmten Grenzen von der Viskosität und /oder von der Temperatur und / oder der Gasbeladung und / oder von anderen physikalischen oder chemischen Zuständen des Fluids ab. Zum Beispiel im Falle von Blut kann die Viskosität aufgrund verschiedener Faktoren, wie zum Beispiel der Zusammensetzung des Bluts, der Beladung mit Gasen, der Temperatur usw. in für die Durchflussmessung durchaus relevanten Grenzen variieren.

Das kann dazu führen, dass die aus den elektrischen Betriebsdaten ermittelten Durchflussmengen mit der Zeit Abweichungen von der tatsächlichen Durchflussmenge zeigen. Das können periodisch oder nicht periodisch schwankende Abweichungen sein, aber auch langfristig sich entwickelte Driftbewegungen sein, so dass nach einer bestimmten Betriebsdauer die aus den Pumpendaten ermittelten Durchflussmengen immer mehr von den tatsächlichen Durchflussmengen abweichen.

Es ist klar, dass auf Grund solcher fehlerhaften Durchflussdaten eine zuverlässige Regelung und / oder Steuerung und / oder Beurteilung des Strömungssystem, vor allem eines solch sensiblen Systems wie eines Ventrikel Unterstützungssystems nicht mehr möglich ist und eventuell sogar fatale Folgen für einen an das System angeschlossenen Patienten haben kann.

Es liegt somit auf der Hand, dass zur Sicherstellung einer zuverlässigen Messung der Durchflussmengen zusätzliche Massnahmen getroffen werden müssen.

Es liegt die Möglichkeit nahe, den Durchfluss zum Beispiel durch eine eigene, möglichst nicht invasive Messapparatur zu bestimmen, die im wesentlichen unempfindlich auf die zuvor beschrieben Schwankungen im System, wie Schwankungen der Temperatur, der Viskosität usw. reagiert, und so auch unter Berücksichtigung der möglichen relevanten Schwankungen im Strömungssystem zuverlässige und verlässliche Messdaten zum Durchfluss des Fluids liefert.

Eine Methode mit der dies im Prinzip möglich ist, besteht in der Messung der Laufzeit eines kurzen Ultraschallimpulses, bzw. von Paketen von Ultraschallwellen, der / die z.B. gegen die Strömungsrichtung des Fluids durch das fliessende Fluid von einem ersten Sender geschickt wird, und von einem gegen die Strömungsrichtung axial versetzen akustischen zweiten Empfänger empfangen wird. In modernen Geräten wird die Laufzeitdifferenz in an sich bekannter Weise mit sogenannten DSP's bestimmt, wobei häufig Kreuzkorrelationsverfahren zum Einsatz kommen.

Dabei kann der zuvor erwähnte zweite Empfänger seinerseits als zweiter Sender betrieben werden, der ebenfalls einen kurzen Ultraschallimpuls bzw. das Paket von Ultraschallwellen diesmal in Strömungsrichtung des Fluids durch das Fluid zu dem ersten Sender schicken, der zum Empfang des Ultraschallimpulses bzw. des Pakets von Ultraschallwellen vom zweiten Sender gleichzeitig als zweiter Empfänger betrieben werden kann.

Die Laufzeit des gegen die Strömung des Fluids gemessenen Ultraschallimpulses bzw. des Pakets von Ultraschallwellen wird verglichen mit derjenigen Laufzeit des Ultraschallimpulses oder mit derjenigen Laufzeit des Pakets von Ultraschallwellen, der / die das strömende Fluid in Strömungsrichtung durchlaufen hat. Aus der Laufzeitdifferenz der beiden Ultraschallimpulse bzw. der Pakete von Ultraschallwellen kann dann in einer dem Fachmann wohlbekannten Weise die Strömungsgeschwindigkeit des Fluids ermittelt werden.

In Fällen, in denen die Strömungsgeschwindigkeit und / oder die Dichte des Fluids in Abhängigkeit von den möglichen Schwankungen der Systemparameter, also den möglichen Schwankungen der Temperatur, der Viskosität usw. nicht in relevanter Weise und nicht signifikant abhängt, kann aus der ermittelten Strömungsgeschwindigkeit des Fluids bei bekannter Geometrie des Strömungssystem bzw. einer Strömungsverbindung, an der die Messung vorgenommen wird, unmittelbar die Durchflussmenge zuverlässig ermittelt werden, ohne dass die vorgenannten Schwankungen das Messergebnis negativ beeinflussen.

Es ist klar, dass als zusätzliches Messsystem nicht nur akustische Systeme zum Einsatz kommen können. Im Prinzip sind natürlich auch mechanische, induktive, kapazitive oder optische Messsysteme geeignet, mit denen die Durchflussmenge über einen geeignete Bewegungsgrösse des Fluids, also zum, Beispiel über dessen Strömungsgeschwindigkeit, geeignet und zuverlässig bestimmbar ist.

Aber vor allem dann, wenn die zur Betreibung des Systems benötigte elektrische Energie nicht in ausreichendem Masse zur Verfügung steht bzw. schwierig zur Verfügung zu stellen ist, müssen entsprechende Nachteile in Bezug die Energieversorgung des Strömungssystems in Kauf genommen werden.

Vor allem die zusätzliche Elektronik die benötigt wird, um das zusätzliche Messsystem zu betreiben und auszuwerten, kann eine erhebliche Menge an zusätzlicher elektrischer Energie verbrauchen, was nicht tolerierbar oder zumindest nur schwer kompensierbar ist.

Ein prominentes Beispiel für ein solches Strömungssystem sind die bereits mehrfach erwähnten Ventrikel Unterstützungssysteme zur Unterstützung eines Blutkreislaufs, im Speziellen zur Unterstützung oder zeitweisen Ersetzung der Herztätigkeit eines Patienten.

Eine in diesem Zusammenhang besonders wichtige Ausführungsform sind portable Systeme, die einem Patienten ein Höchstmass an Bewegungsfreiheit ermöglichen, so dass dieser zum Beispiel nicht mehr ständig ans Bett gefesselt ist, sondern sich mehr oder weniger frei bewegen kann. Das ist besonders in solchen Fällen wichtig, wo der Patient das Ventrikel Unterstützungssystem über längere Zeit, z.B. für mehrere Tage, Wochen oder gar Monate permanent tragen muss, z.B. weil das Herz durch einen durch das Ventrikel Unterstützungssystem bereitgestellten Bypass über längere Zeit entlastet werden muss, in der Hoffnung, dass sich das krankhafte Herz dadurch wieder regeneriert bzw. auf Dauer wieder hergestellt wird, oder aber z.B. bis die Voraussetzungen für eine Herztransplantation hergestellt sind.

Bei solchen portablen Ventrikel Unterstützungssystemen muss der Patient natürlich einen entsprechenden elektrischen Energievorrat, z.B. in Form von Batterien oder Akkumulatoren mit sich führen, da sowohl die Blutpumpe des Systems, als auch die gesamte dazugehörige Steuer- und Auswerteelektronik permanent mit elektrischer Energie versorget werden muss.

Es versteht sich, dass bei einem solchen System die Höhe des Energieverbrauchs eine äusserst kritische Grösse ist, die unter allen Umständen zu minimieren ist.

Somit wirkt sich eine zusätzliche Apparatur inklusive Elektronik zur Bestimmung der Durchflussdaten des Fluids in Bezug auf den Verbrauch von elektrischer Energie negativ aus, so dass zu häufig der mitgeführte elektrische Energiespeicher neu aufgeladen werden muss bzw. ausgetauscht werden muss, ein Aufwand der natürlich aus naheliegenden Gründen möglichst zu minimieren ist. Darüber hinaus wird durch den zusätzlichen Leistungsverbrauch zusätzliche Wärme erzeugt, was in vielen Fällen nicht wünschenswert oder sogar schädlich ist.

Die Aufgabe der Erfindung ist es daher, ein Verfahren zur Kalibrierung einer Durchflussmenge in einem Strömungssystem, insbesondere in einem tragbaren Ventrikel Unterstützungssystem, sowie ein Strömungssystem, im speziellen ein Ventrikel Unterstützungssystem zur Verfügung zu stellen, mit welchem zuverlässig, auch über längere Zeiträume, bis zu Tagen, Wochen oder Monaten, typischerweise bis zu sechs Monaten oder länger, die Durchflussmenge des Fluids durch das Strömungssystem messbar, insbesondere nicht-invasiv messbar ist, wobei gleichzeitig der Verbrauch an elektrischer Energie zur Durchführung der Durchflussmessung minimiert wird.

Die diese Aufgaben in verfahrenstechnischer und apparativer Hinsicht lösenden Gegenstände der Erfindung sind durch die Merkmale des unabhängigen Anspruchs der jeweiligen Kategorie gekennzeichnet.

Die jeweiligen abhängigen Ansprüche beziehen sich auf besonders vorteilhafte Ausführungsformen der Erfindung.

Erfindungsgemäss wird somit ein Verfahren zur Kalibrierung einer Durchflussmessung in einem Strömungssystem vorgeschlagen, wobei ein Fluid durch eine Strömungsverbindung des Strömungssystems befördert wird, und durch die Durchflussmessung eine Durchflussmenge des Fluids aus einem Arbeitsparameter des Strömungssystems bestimmt wird. Erfindungsgemäss wird dabei zu einem vorgegebenen Zeitpunkt mit einem Kalibriersensor die Durchflussmenge des Fluids in einer Kalibriermessung ermittelt, und die Durchflussmessung anhand der Kalibriermessung neu kalibriert.

Wesentlich für die Erfindung ist, dass die eigentliche Durchflussmessung durch Auswertung des Arbeitparameters des Strömungssystems, also zum Beispiel aus dem Speisestrom und / oder aus der Rotordrehzahl einer Pumpe des Systems ermittelt wird. Damit benötigt die eigentliche Durchflussmessung keine zusätzliche Energie und keine zusätzlichen apparativen Einrichtungen, die zur Bestimmung des Durchflusses des Fluids durch das Strömungssystem zum Betrieb ständig mit elektrischer Energie versorgt werden müsste.

Durch die Erfindung wurde vielmehr erstmal erkannt, dass eine mehr oder weniger regelmässige Korrektur der aus dem Arbeitsparameter ermittelten Durchflussdaten völlig ausreicht, um auch langfristig Schwankungen bzw. Abweichungen aufgrund von Änderungen im Strömungssystem, z.B. aufgrund von Änderungen in der Viskosität des Fuids, aufgrund von Temperaturschwankungen oder auf Grund von anderen Veränderungen im System auszugleichen, so dass auch langfristig zuverlässige Daten der Durchflussmengen zur Steuerung und / oder Regelung des Strömungssystems zur Verfügung stehen.

Durch das erfindungsgemässe Verfahren ist also nur noch von Zeit zu Zeit mit dem Kalibriersensor eine kurzzeitige Kalibriermessung der Durchflussmenge des Fluids notwendig. Zwischen den einzelnen kurzzeitigen Kalibriermessungen sind keine Messungen mit dem Kalibriersensor notwendig und die gesamte Elektronik zur Betreibung und Auswertung des Kalibriersensors wird zwischen zwei Kalibriermessungen bevorzugt abgeschaltet, oder in einen Zustand versetzt, in dem der Leistungsverbrauch drastisch herabgesetzt ist. Dadurch lässt sich eine bedeutende Menge an elektrischer Energie sparen, die bei einem speziellen Ausführungsbeispiel eines erfindungsgemässen Strömungssystems bis nahezu 10% des Gesamtenergieverbrauchs ausmachen kann.

Wie bereits erwähnt, wird das Fluid mit einer Pumpe durch das Strömungssystem befördert und die Durchflussmenge aus einem elektrischen Betriebsparameter der Pumpe, insbesondere aus dem elektrischen Antriebstrom und / oder der Drehzahl des Rotors der Pumpe bestimmt. Diese Methode der permanenten Durchflussmessung ist nicht nur besonders energiesparend, da die elektrischen Betriebsparameter in der Antriebselektronik des Motors quasi automatisch zur Verfügung stehen und daher nicht separat ermittelt werden müssen. Auch apparativ ist diese Lösung, und damit wiederum mit Blick auch auf den Energieverbrauch besonders zu bevorzugen, da keinerlei zusätzlichen apparativen Einrichtungen für die permanente Überwachung des Durchflusses des Fluids durch das Strömungssystem vorgesehen werden müssen.

In einem für die Praxis besonders wichtigen Ausführungsbeispiel wird die Pumpe von einem elektrischen Drehantrieb angetrieben, der einen Stator mit einer Statorwicklung und einen im Fluid rotierbaren Rotationskörper umfasst, wobei der Rotationskörper als Rotor des Drehantriebs ausgestaltet ist, und der Rotationskörper bezüglich des Stators berührungslos magnetisch gelagert ist. Der Antrieb und die magnetische Lagerung des Rotors bilden dabei nach dem Prinzip des lagerlosen Motors eine Einheit.

Unter einem lagelosen Motor ist im Rahmen dieser Anmeldung dabei das Prinzip eines Motors mit einem rein magnetisch gelagerten Rotor zu verstehen, wie er z.B. in der EP 1 284 415 A1 oder der WO 96/31934 ausführlich beschrieben ist, deren Inhalt hiermit in diese Anmeldung mit aufgenommen ist. Die berührungsfrei magnetisch gelagerten Motoren dieser Art sind dabei im wesentlichen dadurch gekennzeichnet, dass eine solche Rotationsmaschine einen angetriebenen Rotor und einen elektrischen Motor aufweist, der einen Stator und den antreibenden Rotor umfasst, wobei der Stator auch als elektromagnetisches Lager für den antreibenden Rotor ausgebildet ist und der antreibende Rotor des Elektromotors mit dem angetriebenen Rotor der Rotationsmaschine eine Rotoreinheit, d.h. einen Integralrotor bildet, wobei lediglich ein einziger Stator vorgesehen ist, der den Integralrotor sowohl lagert als auch antreibt.

Als Kalibriersensoren können im Grunde alle aus dem Stand der Technik im Prinzip bekannten geeigneten Sensoren zum Einsatz kommen, wie zum Beispiel ein Differentialdrucksensor und / oder ein Flügelradsensor und / oder ein Sensor mit einem Schwebekörper, und / oder ein optischer oder akustischer Sensor, insbesondere ein Ultraschallsensor. Besonders bevorzugt wird dabei für ein erfindungsgemässes Strömungssystem mindestens ein Paar aus zwei korrespondierenden Ultraschallsensoren, die im speziellen auf piezoelektrischer Basis arbeiten und sowohl als Ultraschallsender als auch als Ultraschallempfänger betreibbar sind. Das entsprechende, an sich bekannte Messprinzip wird in der nachfolgenden Figurenbeschreibung noch ausführlich diskutiert werden.

Wie ebenfalls bereits erwähnt, wird die Durchflussmenge des Fluids kontinuierlich aus dem Arbeitsparameter des Strömungssystems bestimmt, da der Arbeitsparameter, z.B. die Stromaufnahme des Pumpenmotors häufig ohnehin unterbrochen und automatisch zur Verfügung steht. Es versteht sich dabei, dass natürlich auch die Durchflussmessung aus dem Arbeitsparameter nicht unbedingt kontinuierlich erfolgen muss, sondern ebenfalls zeitweise unterbrochen sein kann, wodurch zusätzlich elektrische Energie in der zugehörigen Auswerteelektronik eingespart werden kann.

Die Kalibriermessung mit dem Kalibriersensor erfolgt dagegen in vorgegebenen Zeitabständen, ist zwischen zwei Messungen unterbrochen und wird im Speziellen periodisch, d.h. in gleichen Zeitabständen zur Kalibrierung der Durchflussmessung immer wieder wiederholt.

Der Kalibriersensor kann an eine separate Auswerteeinheit mit einer elektrischen Energieversorgung angeschlossen und so mit elektrischer Energie versorgt werden, und / oder die Auswerteinheit wird wie bereits eingehend erläutert, zwischen zwei Kalibriermessungen abgeschaltet oder in einen energiesparenden Zustand versetzt.

Es versteht sich, dass der Kalibriersensor auch mit der Energieversorgung der Steuerelektronik des Pumpenmotors mit elektrischer Energie versorgt werden kann und / oder die Auswerteeinheit des Kalibriersensors ein integraler Bestandteil der Steuerelektronik des Pumpenmotors sein kann, der zum Beispiel separat abschaltbar ist.

Besonders vorteilhaft einsetzbar ist das erfindungsgemässe Verfahren bei einem Ventrikel Unterstützungssystem zur Unterstützung eines menschlichen oder tierischen Blutkreislaufs, da durch das erfindungsgemässe Verfahren eine beträchtliche Menge an elektrischer Energie eingespart werden kann und im Speziellen die Kalibrierung der Durchflussmessung durch nicht-invasive Verfahren, also z.B. durch ein Ultraschallmessverfahren durchgeführt wird, so dass die eigentliche Messung das Fluid weder chemisch noch physikalisch in irgendeiner Weise negativ beeinflusst wird.

Im Folgenden soll an einem speziellen, für die Praxis besonders wichtigen Ausführungsbeispiel der Vorgang der Kalibrierung gemäss der vorliegenden Erfindung unter Verwendung einer von der Anmelderin entwickelten Herzunterstützenden Blutpumpe illustriert werden.

Zur Reduktion von Wärme und des Energieverbrauchs innerhalb eines Gehäuses, das die elektrische Ansteuerung beherbergt, kann die Flussmesseinheit, die im vorliegenden Beispiel ein MiniDigi-Flow-Board ist, für eine vorgebbare Zeitspanne abgeschaltet werden. In dieser Zeitspanne wird der Fluss durch die Flussbestimmungseinheit des UltraMag-Gerätes überwacht. Da die Flussbestimmungseinheit über längere Zeiträume nicht sehr stabil ist, wird, um einen Drift in der Flussbestimmung zu vermeiden, ein Offset in der Flussbestimmung regelmässig mit Messwerten korrigiert, die das MiniDigi-Flow-Board liefert.

Dabei kann das Abschalten der Flussmesseinheit, also des MiniDigi-Flow-Boards im Prinzip auf zweite Arten erfolgen: periodische Leistungsreduktion, d.h. Übergang des Mini-Digi in einen sogenannten "Sleep-Mode" und Flussbestimmung durch die Flussbestimmungseinheit des UltraMag-Geräts, oder periodische Abschaltung des MiniDigi-Flow-Boards und Flussbestimmung durch die Flussbestimmungseinheit des UltraMag-Geräts.

Dabei ist in vielen Fällen die Variante, in der das MiniDigi abgeschaltet wird, vorzuziehen, da das Abschalten weniger Programmier- und auch weniger apparativen Aufwand erfordert, wodurch dieses Verfahren im Vergleich zu der Variante, in der das MiniDigi in einen Sleep-Mode versetzt wird, oft effektiver und kostengünstiger ist.

In einem für die Praxis sehr wichtigen Beispiel wird das MiniDigi-Flow-Board z.B. für eine Zeitspanne von 10 Minuten abgeschaltet. Die Flussbestimmungseinheit des UltraMag-Geräts wird nur alle 10 Minuten für z.B. 15 Sekunden kurz angeschaltet bzw. aktiviert. Dies gestattet der Flussbestimmungseinheit die Bestimmung eines Tiefpass gefilterten Flusswertes. Dieser Wert wird dann benutzt um den aktuellen Offset der Flussberechnung zu bestimmen.

Wenn die Flussbestimmungseinheit abgeschaltet ist, wird der Fluss berechnet. Jedes mal wenn das MiniDigi-Flow-Board angeschaltet wird, wird die Flussbestimmung kalibriert, um den gemessenen Fluss abzugleichen.

Das zuvor beschriebene Grundkonzept kann der Fachmann natürlich noch variieren und an spezielle Bedürfnisse anpassen. Ausserdem versteht es sich, dass in der Praxis auch die Möglichkeit eines Ausfalls oder z.B. eines Unterbruchs des Durchflusses entsprechend zu berücksichtigen sind.

In einem speziellen Beispiel wird er Fluss alle ca. 2.6 ms berechnet. Daher kann die oben beschriebene Flussberechnung im Verhältnis zur Frequenz der Flussmessung und der Kalibrierfrequenz als quasi kontinuierlich angesehen werden.

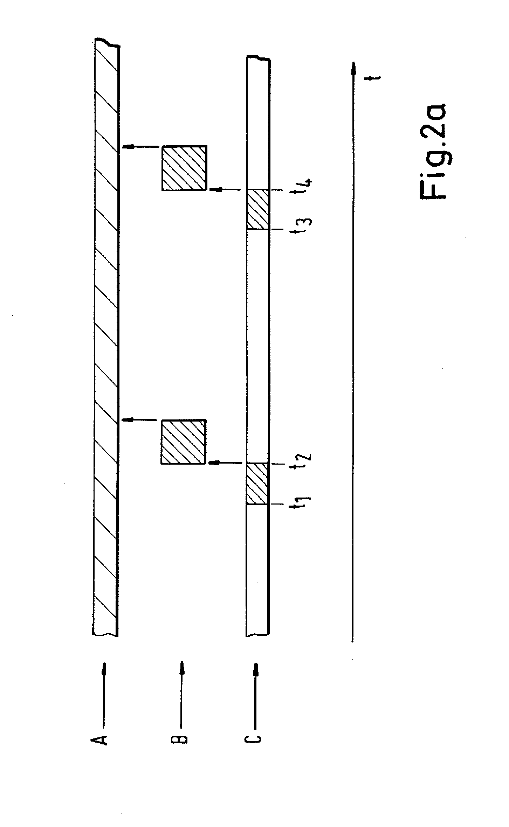

Das zuvor beschriebene Kalibrierverfahren kann daher vereinfacht gemäss Fig. 2a schematisch dargestellt werden.

Der darstellungsgemäss untere Pfeil symbolisiert die Zeitachse, während die entsprechenden Zeitabhängigkeiten der Betriebszustände des MiniDigi-Flow-Boards, in Fig. 2a mit C bezeichnet, der UltraMag-Flusskaibrierung, in Fig. 2a mit B bezeichnet und der UltraMag Flussbestimmungseinheit, welche mit A bezeichnet ist, durch die mit A, B und C bezeichneten Balken angezeigt sind.

In den schraffierten Bereichen ist der zugehörige Betriebszustand ein eingeschalter Zustand, in den nicht-schraffierten Bereichen ist der betreffende Betriebszustand ein ausgeschalteter Zustand.

Darstellungegemäss von links kommend ist das MiniDigi-Flow-Board also zunächst ausgeschaltet. Das heisst, der Fluss mittels der beschriebenen Ultraschallsensoren wird aktuell nicht gemessen.

Dann wir zwischen den Zeitpunkten $t_1$ und $t_2$ das MiniDigi eingeschaltete und die aktuelle Durchflussmenge bestimmt. Nach Abschalten des MiniDigi zum Zeitpunkt $t_2$ wird die Kalibrierung des Offsets berechnet und danach die berechnete Kalibrierung der quasi kontinuierlichen Flussberechnung zur Verfügung gestellt und zur Kalibrierung der Flussberechnung benutzt.

Der ganze Vorgang wiederholt sich dann periodisch, als nächstes, wie in Fig. 1 dargestellt, wieder ab dem Zeitabschnitt zwischen den Zeitpunkten $t_3$ und $t_4$ usw.

Im Beispiel der Fig. 2a erfolgt die Flussberechnung also quasi kontinuierlich alle 2.6 ms, weshalb der Balken A durchgehend schraffiert werden konnte. Die Kalibrierung und die Ultraschallmessung werden jeweils nur alle 10 Minuten durchgeführt, wodurch erfindungsgemäss eine grosse Menge an elektrischer Energie gespart wird.

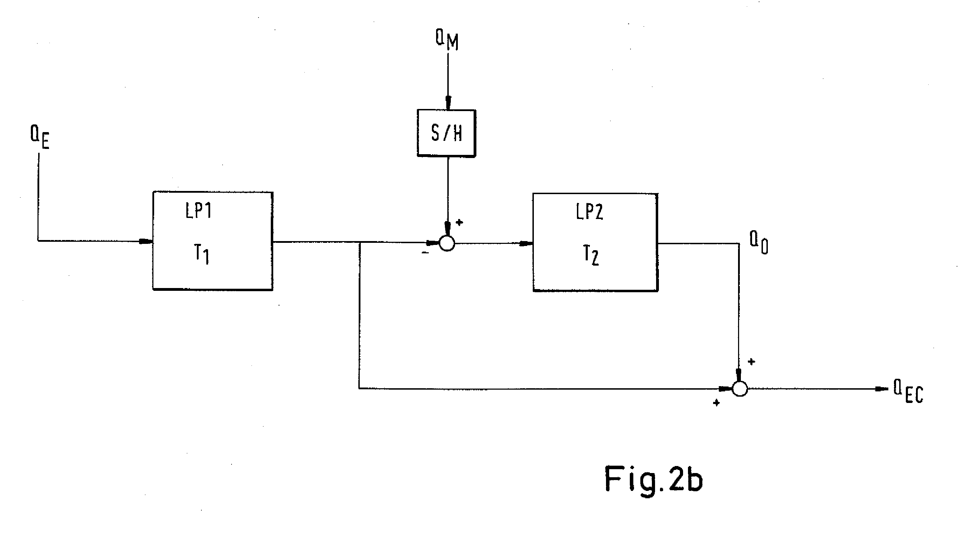

In Fig. 2b ist schliesslich noch der zugehörige Algorithmus in schematischer Weise vereinfacht dargestellt.

$Q_E$ bedeutet dabei den auf Basis der Impeller Geschwindigkeit und des drehmomentbildenden Stroms berechneten Durchfluss. S/H ist ein an sich bekanntes "Sample-and-Hold Glied" mit einer Abtastrate im Minutenbereich, $Q_M$ ist der zum Beispiel alle 10 Minuten mittels der beschriebenen Ultraschalltechnik gemessene Referenzdurchfluss. $Q_O$ ist der Offset Korrekturfaktor und $Q_{EC}$ der kalibrierte Fluss. LP1 bezeichnet einen Tiefpassfilter mit Zeitkonstante $T_1$ im Sekunden- bis Minutenbereich, wobei in einem speziellen Ausführungsbeispiel die Zeitkonstante $T_1$ z.B. $T_1$=10s ist, und LP2 einen Tiefpassfilter mit Zeitkonstante $T_2$ im Sekunden- bis Minutenbereich, wobei im Speziellen z.B. 1min<$T_2$<30min gelten kann.

Da eine Änderung im Hematorcrit des Blutes grundsätzlich zu einer Änderung im Offset führt, kann die Kalibrierung durch eine Offset Kalibrierung bewerkstelligt werden, wie in Fig. 2b schematisch gezeigt. Zusätzlich wird der Offset Korrekturfaktor durch einen Tiefpass gefiltert.

Die Erfindung betrifft weiter ein Strömungssystem zur Durchführung eines erfindungsgemässen Verfahrens.

In einem für die Praxis besonders wichtigen Ausführungsbeispiel ist ein erfindungsgemässes Strömungssystem ein Ventrikel Unterstützungssystem zur Unterstützung eines menschlichen oder tierischen Blutkreislaufs, umfassend eine Pumpe mit einem lagerlosen Motor, eine mit dem lagerlosen Motor Signal verbundene Ansteuereinheit zur Steuerung und / oder Regelung eines Betriebsparameters der Pumpe, wobei die Ansteuereinheit eine Messeinheit zur Bestimmung einer Durchflussmenge von Blut aus einem Arbeitsparameter des lagerlosen Motors umfasst. Erfindungsgemäss ist dabei ein akustischer Kalibriersensor vorgesehen, mit welchem zu einem vorgegebenen Zeitpunkt die Durchflussmenge des Blutes in einer Kalibriermessung ermittelbar ist, und die Durchflussmessung anhand der Kalibriermessung neu kalibrierbar ist. Dabei wird im Betriebszustand die Kalibriermessung mit dem Kalibriersensor in vorgegebenen Zeitabständen wiederholt. Für den Kalibriersensor ist eine Auswerteeinheit vorgesehen, wobei die Auswerteinheit derart ausgestaltet ist, dass sie zwischen zwei Kalibriermessungen abschaltbar ist oder in den energiesparenden Zustand versetzt werden kann.

Besonders bevorzugt ist die Pumpe eine Batterie betriebene Pumpe und /oder die Ansteuereinheit ist eine Batterie betriebene Ansteuereinheit und /oder die Auswerteeinheit ist eine Batterie betriebene Auswerteeinheit.

Dabei ist in einem speziellen Ausführungsbeispiel ein erfindungsgemässes Strömungssystem derart ausgestaltet, dass ein mechanisches Drehmoment und / oder ein Motorstrom zur Bestimmung der Durchflussmenge verwendbar ist.

Im Folgenden wird die Erfindung an Hand der schematischen Zeichnung näher erläutert. Es zeigen:

Fig. 1a    eine Leistungskurve zum Zusammenhang zwischen mechanischer Leistung und Durchfluss;

Fig. 1b    eine Leistungskurve von Blutersatzflüssigkeiten unterschiedlicher Viskosität bei einer konstanten Drehzahl;

Fig. 2a     ein Kalibrierverfahren in schematischer Darstellung;

Fig. 2b     ein Schema eines erfindungegemässen Kalibrieralgorithmus;

Fig. 3      ein erfindungsgemässes Ventrikel Unterstützungssystem;

Fig. 4      ein erstes Ausführungsbeispiel eines Ultraschall Kalibriersensors;

Fig. 5      ein zweites Ausführungsbeispiel gemäss Fig. 4;

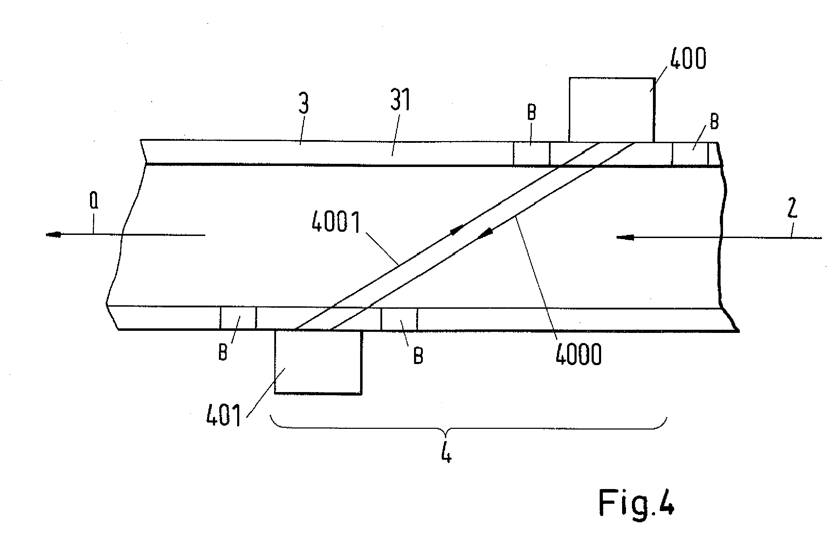

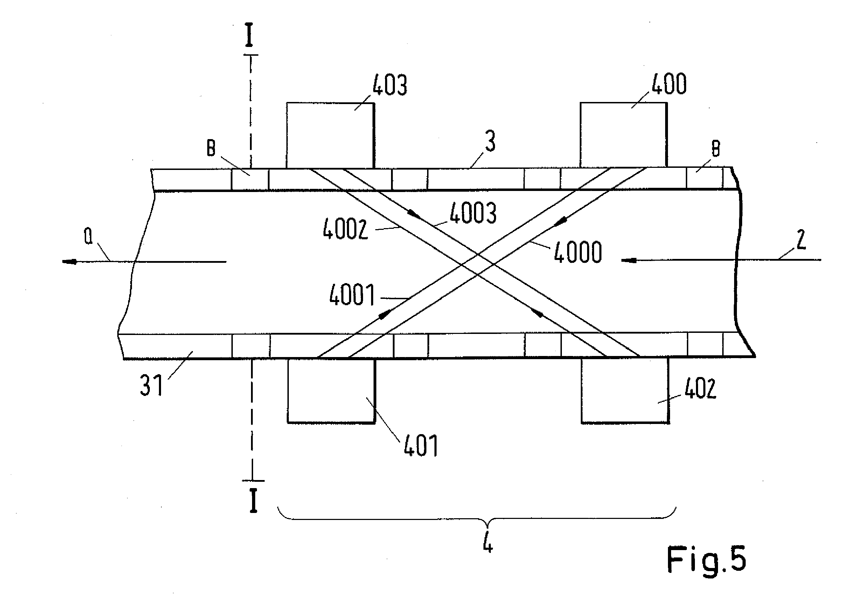

Fig. 4      einen Schnitt entlang der Schnittlinie I - I gemäss Fig. 5.

Die Fig. 1a bis 2b wurde bereits oben ausführlich diskutiert und brauchen daher im folgenden nicht nochmals diskutiert zu werden.

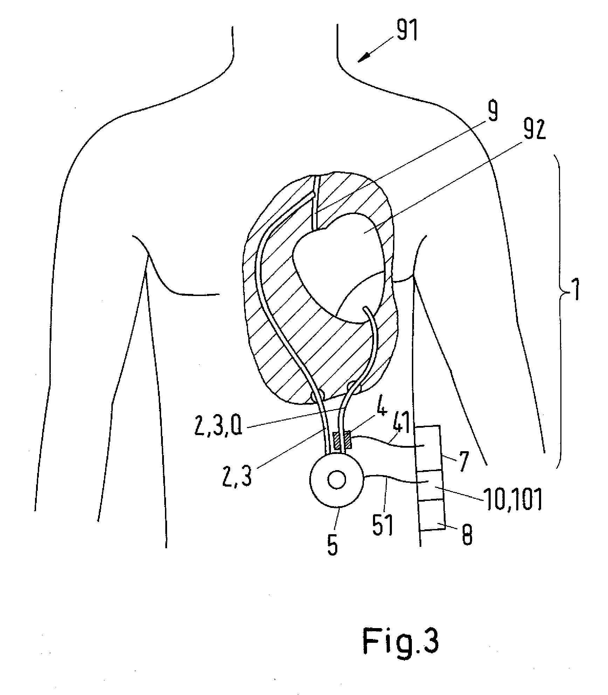

Fig. 3 zeigt in einer schematischen Darstellung ein besonders bevorzugtes Ausführungsbeispiel eines erfindungsgemässen Strömungssystems, das im folgenden gesamthaft mit dem Bezugszeichen 1 bezeichnet wird.

Das Strömungssystem1 der Fig. 3 ist ein portables Ventrikel Unterstützungssystem, das sowohl innerhalb als auch ausserhalb des menschlichen Körpers vorgesehen sein kann und das mit einem Blutkreislauf 9 eines menschlichen Patienten 91 verbunden ist. Alle Systemkomponenten des Ventrikel Unterstützungssystem 1 trägt der Patient 91 am oder im Körper, so dass der Patient 91 völlige Bewegungsfreiheit geniesst. Es versteht sich, dass die Erfindung natürlich nicht auf portable Systeme beschränkt ist, sondern vorteilhaft auch bei stationären Systemen einsetzbar ist. Bestimmte Komponenten des Strömungssystem 1 oder zumindest Teile davon können, müssen aber nicht, Batterie betrieben sein.

Das Ventrikel Unterstützungssystem 1 umfasst eine Blutpumpe 5, im vorliegenden Fall eine Blutpumpe 5, die von einem lagerlosen Motor im Sinne dieser Anmeldung angetrieben wird, die an einem Pumpeneingang und an einem Pumpenausgang über zwei Strömungsverbindungen 3, im vorliegenden Beispiel zwei Herzkanülen 3, in an sich bekannter Weise mit dem Blutkreislauf 9 des Patienten 91 verbunden, so dass zum Beispiel zur Entlastung des Herzens 92 des Patienten 91 Blut 2 über die Strömungsverbindungen 3 der Blutpumpe 5 vom Blutkreislauf 9 zugeführt werden kann und durch die Pumpe 5 unter Erzeugung einer Pumpleistung wieder in den Blutkreislauf 9 zurückgepumpt werden kann. Die Blutpumpe 5 ist über eine elektrische Steuer- und Signalleitung 51 mit einer Ansteuereinheit 10 signalverbunden. Die Ansteuereinheit 10 umfasst eine Messeinheit 101, mit der aus den Betriebsparametern des elektrischen Antriebs der Blutpumpe 5 kontinuierlich durch eine Durchflussmessung eine Durchflussmenge (Q) des Bluts 2 bestimmt wird. Ausserdem wird über die Signalleitung 51 der elektrische Antrieb mit elektrischer Energie und elektrischen Steuersignalen versorgt.

An einer der beiden Strömungsverbindungen 3, die die Blutpumpe 5 mit dem Blutkreislauf 9 zur Beförderung von Blut 2 verbindet, ist ein Ultraschall Kalibriersensor 4 vorgesehen. Dabei ist es für die vorliegende Erfindung unwesentlich, ob der Kalibriersensor 4 am Eingang oder am Ausgang der Blutpumpe 5 vorgesehen ist. Je nach Bauart der Pumpe kann der Kalibriersensor 4 z.B. am Ausgang der Blutpumpe 5 vorgesehen sein, wenn am Ausgang z.B. mehr Raum für die Platzierung des Kalibriersensors 4 zur Verfügung steht. Es versteht ausserdem, dass der Kalibriersensor an jeder geeigneten Stelle der Strömungsverbindung 3 vorgehen sein kann, also nicht unbedingt direkt an oder in der Nähe der Pumpe.

Der Ultraschall Kalibriersensor 4 ist über eine elektrische Verbindung 41 mit einer Auswerteeinheit 7 signalverbunden, die dem Kalibriersensor 4 einerseits mit elektrischer Energie und elektrischen Signalen zur Erzeugung von Ultraschallimpulsen versorgt und andererseits die von dem Kalibriersensor 4 gemessen Laufzeitsignale detektiert und auswertet. Die Auswerteeinheit 4 ist ihrerseits mit der Ansteuereinheit 10 bzw. mit der Messeinheit 101 signalverbunden, so dass mit Hilfe der Kalibrierdaten, die mit dem Kalibriersensor z.B. periodisch gemessen werden, die Durchflussmessung bzw. die Durchflussmenge (Q) des Bluts 2, die aus einem Arbeitsparameter des elektrischen Antriebs der Blutpumpe 5 ermittelt wurde, in an sich bekannter Weise kalibriert, d.h. wenn nötig angepasst werden können.

Die Kalibriermessung mit dem Kalibriersensor 4 wird dabei bevorzugt in periodischen Abständen vorgenommen, z.B. im Abstand von bis zu 30sec, oder im Abstand von bis zu 2 min, in speziellen Fällen kann es sogar ausreichen, die Kalibriermessung nur in Abständen von 10 min oder in noch grösseren Abständen vorzunehmen. Zwischen zwei Kalibriermessungen wird die Auswerteeinheit 7 des Kalibriersensors 4 abgeschaltet, so dass die Standzeit der Energieversorgung 8, im vorliegenden Fall des wieder aufladbaren Akkumulators 8, der das gesamte Ventrikel Unterstützungssystem mit elektrischer Energie versorgt, deutlich erhöht wird und dadurch der Akkumulator 8 deutlich seltener neu aufgeladen werden muss. Zusätzlich wird durch den verringerten Leistungsverbrauch und der daraus resultierenden geringeren Verlustleistung, weniger Wärme generiert.

In Fig. 4 ist ein erstes einfaches Ausführungsbeispiel eines Ultraschall Kalibriersensors 4 schematisch dargestellt. Der Kalibriersensor 4 umfasst einen ersten piezoelektrischen Ultraschallsensor 400 und einen zweiten identischen piezoelektrischen Ultraschallsensor 401, die beide sowohl als Sender als auch Empfänger arbeiten könnten und in axialer Richtung gegeneinander versetzt an einer Aussenwand 31 der Strömungsverbindung 3 vorgesehen sind. Im Beispiel der Fig. 2 fliesst das Fluid 2, z.B. Blut 2 wie durch die Pfeile angedeutet, darstellungsgemäss von rechts nach links durch die Strömungsverbindung 3.

Es werden jetzt entweder gleichzeitig oder kurz aufeinanderfolgend von den beiden Ultraschallsensoren 400, 401 jeweils ein kurzer erster und zweiter Ultraschallimpuls bzw. Pakete von Ultraschallwellen 4000 und 4001 erzeugt. Der erste Ultraschallimpuls 4000 wird vom ersten Ultraschallsensor 400 erzeugt, durchläuft das strömende Fluid 2 und wird vom zweiten Ultraschallsensor 401 detektiert. Analog läuft der zweite Ultraschallimpuls vom zweiten Ultraschallsensor 401 zum ersten Ultraschallsensor 400 und wird von diesem detektiert.

Aus den detektierten Signalen ermittelt die in Fig. 4 nicht dargestellte Auswerteeinheit 7 die Laufzeiten der Ultraschallimpulse 4000 und 4001, die aufgrund der Strömungsgeschwindigkeit des Fluids 2 unterschiedlich sind, weil der erste Ultraschallimpuls 4000 eine Geschwindigkeitskomponente in Strömungsrichtung des Fluids hat und der zweite Ultraschallimpuls 4001 eine Geschwindigkeitskomponente entgegen der Strömungsrichtung des Fluids 2 hat. Aus der Laufzeitdifferenz der beiden Ultraschallimpulse 4000 und 4001 lässt sich dann in an sich bekannter Weise die tatsächliche Durchflussmenge (Q) des Fluids 2 bestimmen und damit die Durchflussmessung, die aus den Arbeitsparametern des Strömungssystems gewonnen wurde, kalibrieren, also falls notwendig korrigieren.

Damit die Messung nicht durch Reflexionen des Ultraschalls in der Aussenwand 31 der Strömungsverbindung 3 gestört oder verfälscht wird, können in der Aussenwand 31 Schallbarrieren B vorgesehen sein, die in der Aussenwand 31 umherlaufende akustische Signale brechen bzw. dämpfen, so dass diese im wesentlichen nicht mehr von den Ultraschallsensoren 400, 401 detektiert werden können.

In Fig. 3 ist schematisch ein zweites Ausführungsbeispiel gemäss Fig. 4 dargestellt, das sich von dem Beispiel der Fig. 4 nur dadurch unterscheidet, dass zusätzlich zu dem Sensorpaar 400 und 401 noch ein zweites identisches Sensorpaar 402 und 403 vorgesehen ist, das völlig analog zu dem Sensorpaar 400 und 4001 arbeitet und kurze Ultraschallimpulse 4002 und 4003 austauscht und der Auswerteeinheit 7 zur Auswertung zuführt.

Der Vorteil des Kalibriersensors 4 gemäss Fig. 5 gegenüber dem etwas einfacher aufgebauten Sensor der Fig. 4 liegt darin, dass mit dem Sensor der Fig. 5 eine höhere Auslösung und / oder eine höhere Genauigkeit in der Kalibriermessung erzielbar ist.

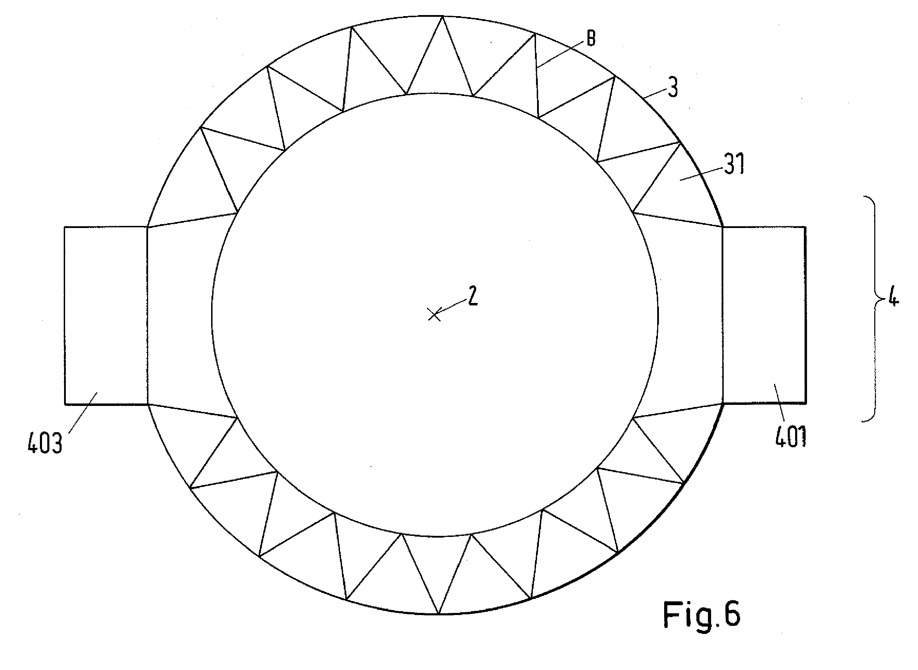

In Fig. 6 ist schliesslich ein Schnitt entlang der Schnittlinie I - I gemäss Fig. 5 dargestellt, der exemplarisch die Funktionsweise und die mögliche Ausgestaltung von Signalbarrieren B in der Aussenwand 31 der Strömungsverbindung illustrieren soll. Die Signalbarrieren B können z.B. Aussparungen, eingearbeitete Unregelmässigkeiten im Material der Aussenwand 31 oder andere gezielt eingebrachte Störungen sind, die in der Aussenwand 31 umlaufende Ultraschallimpulse reflektieren, brechen und damit letztendlich so stark dämpfen, dass sie nicht mehr zu den Sensoren 400, 401, 402, 403 gelangen können, bzw. von diesen nicht mehr als zu verwertende Signale registriert werden.

Es versteht sich, dass die im Rahmen dieser Anmeldung diskutierten Ausführungsbeispiele lediglich exemplarisch zu verstehen sind und insbesondere auch alle geeigneten Kombinationen und dem Fachmann wohlbekannte einfache Weiterentwicklungen von der Erfindung erfasst sind.


**Patentansprüche**

1. Verfahren zur Kalibrierung einer Durchflussmessung in einem Strömungssystem (1), wobei ein Fluid (2) durch eine Strömungsverbindung (3) des Strömungssystems (1) befördert wird, und durch die Durchflussmessung eine Durchflussmenge (Q) des Fluids (2) aus einem Arbeitsparameter des Strömungssystems (1) bestimmt wird, **dadurch gekennzeichnet, dass** zu einem vorgegebenen Zeitpunkt mit einem Kalibriersensor (4) die Durchflussmenge (Q) des Fluids (2) in einer Kalibriermessung ermittelt wird, und die Durchflussmessung anhand der Kalibriermessung neu kalibriert wird.

2. Verfahren nach Anspruch 1, wobei das Fluid mit einer Pumpe (5) durch das Strömungssystem (1) befördert wird und die Durchflussmenge (Q) aus einem elektrischen Betriebsparameter der Pumpe (5), insbesondere aus dem elektrischen Antriebstrom der Pumpe (5) und /oder aus einer Rotordrehzahl (ω) bestimmt wird.

3. Verfahren nach Anspruch 2, wobei die Pumpe (5) von einem elektrischen Drehantrieb (6), der einen Stator mit einer Statorwicklung und einen im Fluid rotierbaren Rotationskörper umfasst, wobei der Rotationskörper als Rotor des Drehantriebs (6) ausgestaltet ist, und der Rotationskörper bezüglich des Stators berührungslos magnetisch gelagert ist, wobei der Antrieb und die magnetische Lagerung des Rotors nach dem Prinzip des lagerlosen Motors eine Einheit bilden.

4. Verfahren nach einem der vorangehenden Ansprüche, wobei der Kalibriersensor (4) ein Differentialdrucksensor (4) und / oder ein Flügelradsensor (4) und / oder ein Sensor (4) mit einem Schwebekörper, und / oder ein akustischer Sensor (4), insbesondere ein Ultraschallsensor (4) ist und / oder ein anderer Durchflusssensor (4) ist.

5. Verfahren nach einem der vorangehenden Ansprüche, wobei die Durchflussmenge (Q) des Fluids (2) kontinuierlich aus dem Arbeitsparameter bestimmt wird.

6. Verfahren nach einem der vorangehenden Ansprüche, wobei die Kalibriermessung mit dem Kalibriersensor (4) in vorgegebenen Zeitabständen, insbesondere periodisch wiederholt wird.

7. Verfahren nach einem der vorangehenden Ansprüche wobei der Kalibriersensor (4) an eine Auswerteeinheit (7) mit einer elektrischen Energieversorgung (8) angeschlossen und mit elektrischer Energie versorgt wird, und / oder die Auswerteinheit (7) zwischen zwei Kalibriermessungen abgeschaltet wird.

8. Verfahren nach einem der vorangehenden Ansprüche, wobei das Strömungssystem (1) ein Ventrikel Unterstützungssystem (1) zur Unterstützung eines menschlichen oder tierischen Blutkreislaufs (9) ist.

9. Strömungssystem zur Durchführung eines Verfahrens nach einem der vorangehenden Ansprüche.

10. Strömungssystem nach Anspruch 9, wobei das Strömungssystem ein Ventrikel Unterstützungssystem zur Unterstützung eines menschlichen oder tierischen Blutkreislaufs (9) ist, umfassend eine Pumpe (5) mit einem lagerlosen Motor, eine mit dem lagerlosen Motor signalverbundene Ansteuereinheit (10) zur Steuerung und / oder Regelung eines Betriebsparameters der Pumpe (5), wobei die Ansteuereinheit (10) eine Messeinheit (101) zur Bestimmung einer Durchflussmenge (Q) von Blut (2) aus einem Arbeitsparameter des lagerlosen Motors umfasst, **dadurch gekennzeichnet, dass** ein akustischer Kalibriersensor (4) vorgesehen ist, mit dem zu einem vorgegebenen Zeitpunkt die Durchflussmenge (Q) des Blutes (2) in einer Kalibriermessung ermittelbar ist, und die Durchflussmessung anhand der Kalibriermessung neu kalibrierbar ist, wobei im Betriebszustand die Kalibriermessung mit dem Kalibriersensor (4) in vorgegebenen Zeitabständen wiederholt wird und für den Kalibriersensor (4) eine Auswerteeinheit (7) vorgesehen ist und die Auswerteinheit (7) derart ausgestaltet ist, dass sie zwischen zwei Kalibriermessungen abschaltbar ist.

11. Strömungssystem nach einem der Ansprüche 9 oder 10, wobei die Pumpe (5) eine Batterie betriebene Pumpe (5) ist und / oder die Ansteuereinheit (10) eine Batterie betriebene Ansteuereinheit (10) ist und / oder die Auswerteeinheit (7) eine Batterie betriebene Auswerteeinheit (7) ist.

12. Strömungssystem nach einem der Ansprüche 9 bis 11, wobei das Strömungssystem derart ausgestaltet ist, dass ein mechanisches Drehmoment ($M_m$) und / oder ein Motorstrom zur Bestimmung der Durchflussmenge (Q) verwendbar ist.

Fig.1a

Fig.1b

A ⟶

B ⟶

C ⟶

$t_1$  $t_2$   $t_3$  $t_4$

t

Fig.2a

Fig.2b

Fig.3

Fig.4

Fig.5

Fig.6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1284415 A1

- WO 9631934 A

**In der Beschreibung aufgeführte Nicht-Patentliteratur**

- **JÜRGEN HAHN.** Sensorlose Bestimmung der Prozessgrössen magnetisch gelagerter Blutpumpen. *Dissertation ETH Zürich,* 2002